# EUROPÄISCHE PATENTSCHRIFT

(11) **EP 2 583 716 B1**
(45) Veröffentlichungstag und Bekanntmachung des Hinweises auf die Patenterteilung: **29.07.2015**
(21) Anmeldenummer: 11185623.3
(22) Anmeldetag: 18.10.2011
(51) Int. Cl.: A61M 39/28

(54) **Infusionsschlauchklemme für eine Infusionspumpe sowie Verfahren zum Verwenden der Infusionsschlauchklemme**
Infusion hose clamp for a infusionpump and method for use of the same
Pince de serrage d'un tube à perfusion pour utilisation dans une pompe à perfusion et procédé d'utilisation de la pince de serrage

(43) Veröffentlichungstag der Anmeldung: 24.04.2013
(73) Patentinhaber: B. Braun Melsungen AG, 34212 Melsungen (DE)
(72) Erfinder: Roth, Stefan, 34212 Melsungen (DE); Gerlach, Hans-Josef, 34431 Marsberg (DE); Scherer, Jörg, 73432 Aalen (DE); Huwyler, Willy, 9330 Cham (CH); Pfyl, Marc, 8852 Altendorf (CH)
(74) Vertreter: Winter, Brandl, Fürniss, Hübner, Röss, Kaiser, Polte - Partnerschaft mbB

(56) Entgegenhaltungen:
- EP-A1- 2 332 611
- EP-A2- 1 000 633
- WO-A1-2007/112500
- WO-A1-2010/149187
- FR-A1- 2 920 513
- GB-A- 2 439 325

## Beschreibung

Die Erfindung betrifft zum einen eine Infusionsschlauchklemme zum Einsetzen in eine Infusionspumpe umfassend einen Grundkörper mit einem Infusionspumpenaufnahmebereich, mit einem ersten Klemmschenkel sowie mit einem zweiten Klemmschenkel, wobei die beiden Klemmschenkel an einem ersten Infusionsschlauchklemmenende schwenkbar miteinander verbunden sind.

Zum anderen betrifft die Erfindung ein Verfahren zum Verwenden einer Infusionsschlauchklemme an einer Infusionspumpe, bei welchem ein erster Klemmschenkel und ein zweiter Klemmschenkel der Infusionsschlauchklemme um einen Infusionsschlauchklemmenendbereich herum aufeinander zu geschwenkt werden, um hierbei einen Infusionsschlauch abquetschen zu können.

Darüber hinaus betrifft die Erfindung eine Infusionspumpe mit einer Aufnahme für eine schlauchseitig angeordnete Infusionsschlauchklemme.

Insbesondere gattungsgemäße schlauchseitige Schnapp- oder Sicherheitsklemmen, kurz auch FFC (free flow clamp) genannt, sind aus dem Stand der Technik vielfältig bekannt. Diese befinden sich oftmals im Rahmen eines Infusionssets bereits an einem Infusionsschlauch, an welchem sie entweder einen Durchfluss eines Infusionsfluids durch den Infusionsschlauch hindurch ermöglichen oder unterbinden sollen. Dies betrifft sowohl einen Zustand vor oder nach einer Montage in einer entsprechenden Infusionspumpe als auch während eines Montagezustandes in die Infusionspumpe. In der Regel ist die schlauchseitige Sicherheitsklemme vor und nach ihrer Montage geschlossen, während sie je nach Infusionspumpenbetriebszustand etwa hinsichtlich einer Infusionsgabe automatisch geöffnet werden kann. Zum Einlegen des die Sicherheitsklemme umfassenden Infusionsschlauchs wird nahezu immer eine Pumpenklappe einer Infusionspumpe geöffnet, um hierdurch Zugang zu einer Infusionsschlauchführung und insbesondere zu einer passenden Sicherheitsklemmenaufnahme zu erhalten.

Im Allgemeinen wird eine derartige schlauchseitige Sicherheitsklemme meist durch eine manuelle Betätigung einer Pumpenklappe einer Infusionspumpe zwangsgesteuert respektive bei einem Öffnen oder Schließen der Pumpenklappe automatisch geschlossen, um einen ungewollten Durchfluss des Infusionsfluids durch den Infusionsschlauch zu unterbinden. Hierbei kann es sein, dass die schlauchseitige Sicherheitsklemme zuerst manuell geschlossen werden muss, bevor die Pumpenklappe ordnungsgemäß geschlossen werden kann. Oftmals verhält es sich aber auch so, dass die schlauchseitige Sicherheitsklemme bereits während des Einlegens geschlossen wird, dann aber mit dem Schließen der Pumpenklappe sogleich wieder geöffnet wird. Eine nach dem Schließen der Pumpenklappe geschlossene schlauchseitige Sicherheitsklemme kann mittels eines Aktuators der Infusionspumpe für den Pumpenbetrieb automatisch wieder geöffnet werden. Neben Infusionspumpen mit einer derartigen Auf-Funktion existieren weitere Infusionspumpen mit einem zusätzlichen Aktuator für eine entsprechende Zu-Funktion zum Schließen der schlauchseitigen Sicherheitsklemme. Darüber hinaus gehören Infusionspumpen zum Stand der Technik, die abgesehen von einer solchen schlauchseitigen Sicherheitsklemme zusätzlich noch eine Geräteklemme mit einer aktuatorischen Auf- und Zu-Funktion besitzen. Die meisten schlauchseitigen Sicherheitsklemmen bauen jedoch immer noch recht aufwendig oder es wird zusätzlich noch eine Geräteklemme an einer Infusionspumpe benötigt, um einen betriebssicheren Infusionsfluidflussstopp innerhalb eines Infusionsschlauchs gewährleisten zu können.

Es ist Aufgabe der Erfindung speziell gattungsgemäße Infusionsschlauchklemmen derart weiterzuentwickeln, dass sowohl eine Herstellung eines entsprechenden Infusionssets umfassend einen Infusionsschlauch als auch eine Verwendung derartiger Infusionsschlauchklemmen an einer Infusionspumpe wesentlich einfacher und betriebssicherer wird.

Die Aufgabe der Erfindung wird von einer Infusionsschlauchklemme nach Anspruch 1 zum Einsetzen in eine Infusionspumpe umfassend einen Grundkörper mit einem Infusionspurnpenaufnahmebereich, mit einem ersten Klemmschenkel sowie mit einem zweiten Klemmschenkel gelöst, wobei die beiden Klemmschenkel an einem ersten Infusionsschlauchklemmenende schwenkbar miteinander verbunden sind, und wobei die Infusionsschlauchklemme sich durch eine Schnappeinrichtung zum sicheren Positionieren der beiden Klemmschenkel in mehr als einer Lage zueinander auszeichnet.

Vorteilhafter Weise kann die vorliegende Infusionsschlauchklemme mittels der Schnappeinrichtung betriebssicher in wenigstens zwei verschiedenen Einschnapplagen gehalten werden.

Der Begriff "Schnappeinrichtung" beschreibt im Sinne der Erfindung jegliche Einrichtungen, mittels welchen die beiden Klemmschenkel in verschiedenen Lagen zueinander gehalten bzw. positioniert werden können.

Vorteilhafter Weise weist die Schnappeinrichtung hierbei ein unlösbares Rastelement und ein lösbares Rastelement auf, wobei das wenigstens eine Schnappelement das unlösbare Rastelement nur einmal in eine erste Richtung passieren kann, und wobei das wenigstens eine Schnappelement das lösbare Rastelement sowohl in die erste Richtung als auch in eine der ersten Richtung entgegengesetzten zweiten Richtung passieren kann.

Der Begriff "lösbares Rastelement" beschreibt ein gegenüber dem Schnappelement weiter auslenkbares Rastelement als hinsichtlich des "unlösbaren Rastelements", wie nachstehend noch erläutert ist, sodass das Schnappelement das lösbare Rastelement in zwei Richtungen passieren kann.

Die beiden Klemmschenkel sind an dem ersten Infusionsschlauchklemmenende idealerweise untrennbar miteinander verbunden. Vorteilhafter Weise ist die vorliegende Infusionsschlauchklemme als einteiliges Spritzgußteil hergestellt.

Die vorliegende Infusionsschlauchklemme ist im Sinne der Erfindung schlauchseitig angeordnet und unterscheidet sich insofern von infusionspumpenseitig verbauten Geräteklemmen. Jedoch ist die vorliegende schlauchseitige Infusionsschlauchklemme zum Einsetzen in eine Infusionspumpe geeignet und sie weist dementsprechend einen Infusionspumpenaufnahmebereich auf, mittels welchem die Infusionsschlauchklemme in einer korrespondierenden infusionspumpenseitigen Aufnahme anordenbar ist. Hierdurch unterscheidet sich die vorliegende Infusionsschlauchklemme von sonstigen Schlauchklemmen.

Um vorteilhaft in einer Infusionspumpe verwendet werden zu können, weist die Infusionsschlauchklemme vorzugsweise eine Betätigungsflanke für einen Infusionspumpenriegel auf.

Bei der derartigen Infusionspumpe handelt es sich vorzugsweise um eine Schlauchpumpe. Da gattungsgemäße Infusionspumpen aus dem Stand der Technik gut bekannt sind, wird vorliegend nicht weiter hierauf eingegangen.

Die Infusionsschlauchklemme weist vorteilhafter Weise einen Herstellzustand auf, bei welchem die beiden Klemmschenkel an dem ersten Infusionsschlauchklemmenende nur einseitig vorzugsweise untrennbar miteinander verbunden sind. Schnappeinrichtungsseitig sind die beiden Klemmschenkel noch nicht miteinander verbunden, sodass ein Infusionsschlauch verfahrenstechnisch außerordentlich einfach in die Infusionsschlauchklemme bzw. in eine Quetscheinrichtung für den Infusionsschlauch eingelegt werden kann. Hierbei entfällt ein aufwendigeres Einfädeln des Infusionsschlauches in die Infusionsschlauchklemme, wodurch sich die Herstellung und Bereitstellung insbesondere eines Infusionssets wesentlich einfacher gestalten lässt.

Eine bevorzugte Ausführungsvariante sieht vor, dass die Schnappeinrichtung jeweils einen Betriebszustand mit einer Öffenstellung, insbesondere einer Quetscheinrichtung zum Quetschen des Infusionsschlauches, und mit einer Quetschstellung, insbesondere der Quetscheinrichtung zum Quetschen des Infusionsschlauches, aufweist, bei welcher wenigstens ein Schnappelement der Schnappeinrichtung in der Öffenstellung an einem ersten Rastelement und in der Quetschstellung an einem zweiten Rastelement eingerastet anordenbar ist.

Vorteilhaft ist es weiter, wenn die beiden Rastelemente in Reihe geschaltet an dem zweiten Klemmschenkel angeordnet sind. Hierdurch kann speziell die Bauhöhe der Schnappeinrichtung gering gehalten werden. Der Begriff "in Reihe" bezieht sich im Sinne der Erfindung auf die Bewegungsrichtung der beiden Klemmschenkel.

Vorzugsweise ist eines der Rastelemente räumlich immer näher an dem ersten Klemmschenkel angeordnet als das andere der Rastelemente.

Die Quetscheinrichtung kann im einfachsten Fall von zwei lediglich stabförmigen Klemmschenkeln ausgestaltet sein. Vorzugsweise weist die Quetscheinrichtung eine Quetschkante an dem ersten Klemmschenkel und eine Quetschkantenaufnahme an dem zweiten Klemmschenkel auf, wobei die Quetscheinrichtung in einer ersten Einschnapplage des Schnappverschlusses noch oder wieder geöffnet und in wenigstens einer weiteren Einschnapplage des Schnappverschlusses geschlossen ist.

Es versteht sich, dass die Schnappeinrichtung vielfältig ausgestaltet sein kann. Beispielsweise befinden sich ein erstes Schnappelement der Schnappeinrichtung an dem ersten Klemmschenkel und ein hiermit korrespondierendes Rastelement der Schnappeinrichtung an dem zweiten Klemmschenkel, während ein weiteres Schnappelement der Schnappeinrichtung an dem zweiten Klemmschenkel und ein hiermit korrespondierendes weiteres Rastelement der Schnappeinrichtung an dem ersten Klemmschenkel angeordnet ist.

Die Infusionsschlauchklemme kann konstruktiv jedoch besonders einfach und platzsparend realisiert werden, wenn die Schnappeinrichtung wenigstens ein dem ersten Klemmschenkel zugeordnetes Schnappelement und wenigstens zwei dem zweiten Klemmschenkel zugeordnete Rastelemente zum Einrasten des wenigstens einen Schnappelements aufweist.

Vorteilhafter Weise ist die Schnappeinrichtung an einem dem ersten Infusionsschlauchklemmenende beabstandeten Bereich angeordnet, sodass zwischen der Schnappeinrichtung und dem ersten Infusionsschlauchklemmenende genügend Bauraum für eine Ausgestaltung einer betriebssicher funktionierenden Abquetschung und Führung des Infusionsschlauches verbleibt.

Baulich kann die vorliegende Infusionsschlauchklemme weiter vereinfacht werden, wenn wenigstens ein Schnappelement und wenigstens zwei Rastelemente der Schnappeinrichtung derart zueinander angeordnet sind, dass beim Schließen der Schnappeinrichtung das Schnappelement zuerst mit dem einen der beiden Rastelemente verrastet bevor das Schnappelement mit dem anderen der beiden Rastelemente verrastbar ist.

Darüber hinaus wird die Aufgabe der Erfindung auch von einem Verfahren nach Anspruch 9 zum Verwenden einer Infusionsschlauchklemme an einer Infusionspumpe gelöst, bei welchem ein erster Klemmschenkel und ein zweiter Klemmschenkel der Infusionsschlauchklemme um einen Infusionsschlauchklemmenendbereich herum aufeinander zu geschwenkt werden, um hierbei einen Infusionsschlauch abquetschen zu können, wobei sich das Verfahren des Weiteren dadurch auszeichnet, dass eine Schnappeinrichtung mit wenigstens einem Schnappelement mit einem ersten Rastelement in eine erste Einschnapplage hinein verrastet wird, in welcher der Infusionsschlauch zwischen den beiden Klemmschenkeln gehalten wird bzw. ist, und dass die Schnappeinrichtung mit dem wenigstens einen Schnappelement oder mit einem weiteren Schnappelement mit einem zweiten Rastelement in eine zweite Einschnapplage hinein verrastet wird, in welcher der Infusionsschlauch abgequetscht wird bzw. ist.

Vorteilhafter Weise wird die Schnappeinrichtung in die erste Einschnapplage überführt, sobald der Infusionsschlauch in die Infusionsschlauchklemme, vorzugsweise quer zur Längserstreckung der beiden Klemmschenkel, eingelegt ist. Somit setzt die Schnappeinrichtung sogleich eine Verliersicherung um, da der Infusionsschlauch radial allseits von der Infusionsschlauchklemme umgeben ist. Infolgedessen kann die Infusionsschauchklemme ohne Zerstörung des Infusionsschlauchs oder der Infusionsschlauchklemme selbst nicht mehr von dem Infusionsschlauch entfernt werden.

Insofern ist es vorteilhaft, wenn die Infusionsschlauchklemme zumindest in einem Betriebszustand eine radial allseits geschlossene Schlauchführung mit einer quer zu der Längserstreckung der beiden Klemmschenkel angeordneten Schlauchführungsrichtung aufweist.

Idealerweise wird die Schnappeinrichtung in die zweite Einschnapplage überführt, sobald sie ordnungsgemäß in die Infusionspumpe eingesetzt wird. Hierdurch ist die Gefahr ausgeschlossen, dass einem Patienten unbeabsichtigt ein Infusionsmittel zugeführt wird, sofern dieser bereits mit dem Infusionsschlauch verbunden ist.

Bei einer bevorzugten Verfahrensvariante wird zum Wiederöffnen des Infusionsschlauchs das zweite Rastelement vorzugsweise derart ausgelenkt, dass die Schnappeinrichtung von der zweiten Einschnapplage in die erste Einschnapplage zurückkehrt.

Zum Öffnen einer entsprechenden Quetscheinrichtung wird also das zweite Rastelement der Infusionsschlauchklemme ausgelenkt, wodurch ein zuvor an dem zweiten Rastelement eingerastetes Schnappelement freigegeben wird.

Zum Auslenken bzw. Lösen eines der beiden Rastelemente ist es vorteilhaft, wenn eines der beiden Rastelemente eine Betätigungsfläche für einen Aktuator einer Infusionsschlauchklemmenbetätigung der Infusionspumpe aufweist, wobei insbesondere das zweite der beiden Rastelemente weiter auslenkbar ausgestaltet ist als das erste der beiden Rastelemente.

Nach einem weiteren Aspekt der Erfindung wird die vorliegende Aufgabe auch von einer Infusionspumpe mit einer Aufnahme für eine schlauchseitig angeordnete Infusionsschlauchklemme und mit einem Aktuator zum Betätigen der schlauchseitigen Infusionsschlauchklemme gelöst, welche sich durch eine Infusionsschlauchklemme nach einem der hier beschriebenen Merkmale auszeichnet, da die Infusionspumpe einfacher konstruiert sein kann als bisher üblich.

Insbesondere benötigt sie im Wesentlichen lediglich eine entsprechend ausgestaltete Aufnahme zum Aufnehmen der vorliegenden Infusionsschlauchklemme bzw. eine Aufnahme für den Infusionspumpenaufnahmebereich der Infusionsschlauchklemme.

Vorteilhafter Weise können auf Basis der vorliegenden Infusionsschlauchklemme eine neue Generation von Infusionspumpen sowie entsprechende Erweiterungskomponenten und entsprechendes Zubehör bereitgestellt werden.

Die Infusionsschlauchklemme ist idealerweise einteilig und sie besitzt ein nicht lösbares und ein lösbares Rastelement. Durch ein manuelles Schließen bzw. Verriegeln einer Pumpenklappe einer Infusionspumpe wird die Infusionsschlauchklemme geschlossen. Wird das lösbare Rastelement ausgelenkt, wechselt die Infusionsschlauchklemme von einer geschlossenen in eine geöffnete Position. Mit Hilfe eines pumpenseitigen Aktuators kann das lösbare Rastelement der Infusionsschlauchklemme, nur bei geschlossener Pumpenklappe, ausgelenkt werden. Die Infusionsschlauchklemme öffnet sich hierbei. Durch ein manuelles Entriegeln der Pumpenklappe wird die Infusionsschlauchklemme geschlossen, bevor die Pumpenklappe geöffnet ist. Die Infusionsschlauchklemme muss vorteilhafter Weise während der Herstellung nicht mehr auf die Leitung bzw. auf den Infusionsschlauch aufgefädelt werden, sondern sie wird einfach aufgeklipst.

Darüber hinaus ergeben sich mittels der vorliegenden Erfindung im Wesentlichen noch folgende Vorteile:
- Ein Free-Flow-Schutz wird vollumfänglich über die schlauchseitige (setbased) FFC erzielt.
- Es ist keine geräteseitige FFC notwendig.
- Beim Schließen und Öffnen der manuell verriegelten Pumpenklappe wird über eine mechanische Zwangssteuerung die FFC immer geschlossen. -> Sicherheitsmerkmal
- Für den Pumpenbetrieb öffnet die Infusionspumpe die schlauchseitige FFC aktuatorisch.
- Ein aktuatorisches Schließen ist nicht erforderlich.
- Der infusionspumpenseitige Aktuator weist nur eine Auf-Funktion auf.
- Es wird durch die Infusionspumpe selber sichergestellt, dass die Infusionspumpe nicht ohne eine korrekt eingelegte FFC in Betrieb genommen werden kann. -> Sicherheitsmerkmal
- Die Verwendung von Infusionssets ohne FFC ist nicht möglich. -> Sicherheitsmerkmal
- Die schlauchseitige FFC ist einteilig aufgebaut.
- Einfachere Montage in der Produktion durch aufklipsen anstatt auffädeln.
- Zum Schutz vor einer unerwünschten Manipulation ist die FFC nicht ohne Beschädigung des Schlauchs oder der FFC selbst, beispielsweise durch Bruch des nicht lösbaren Rastelements entfernbar. -> Sicherheitsmerkmal

Weitere Vorteile, Ziele und Eigenschaften vorliegender Erfindung werden anhand anliegender Zeichnung und nachfolgender Beschreibung erläutert, in welchen beispielhaft erfindungsgemäße Infusionsschlauchklemmen dargestellt und beschrieben sind. In der Zeichnung zeigen:
- Fig. 1: schematisch eine Ansicht eines ersten Ausführungsbeispiels einer Infusionsschlauchklemme mit einer Schnappeinrichtung umfassend ein einziges Schnappelement und zwei Rastelemente hierfür an einer Infusionspumpe angeordnet;
- Fig. 2: schematisch eine Ansicht der Infusionsschlauchklemme aus der Figur 1 in einem ersten Betriebszustand hinsichtlich einer Quetschstellung;
- Fig. 3: schematisch eine weitere Ansicht der Infusionsschlauchklemme aus den Figuren 1 und 2 in einem weiteren Betriebszustand hinsichtlich einer Öffenstellung;
- Fig. 4: schematisch eine Ansicht eines zweiten Ausführungsbeispiels einer anderen Infusionsschlauchklemme in einem Herstellzustand;
- Fig. 5: schematisch eine weitere Ansicht der Infusionsschlauchklemme aus der Figur 4 in einem ersten Betriebszustand hinsichtlich einer Öffenstellung; und
- Fig. 6: schematisch eine Ansicht der Infusionsschlauchklemme aus den Figuren 4 und 5 in einem weiteren Betriebszustand hinsichtlich einer Quetschstellung.

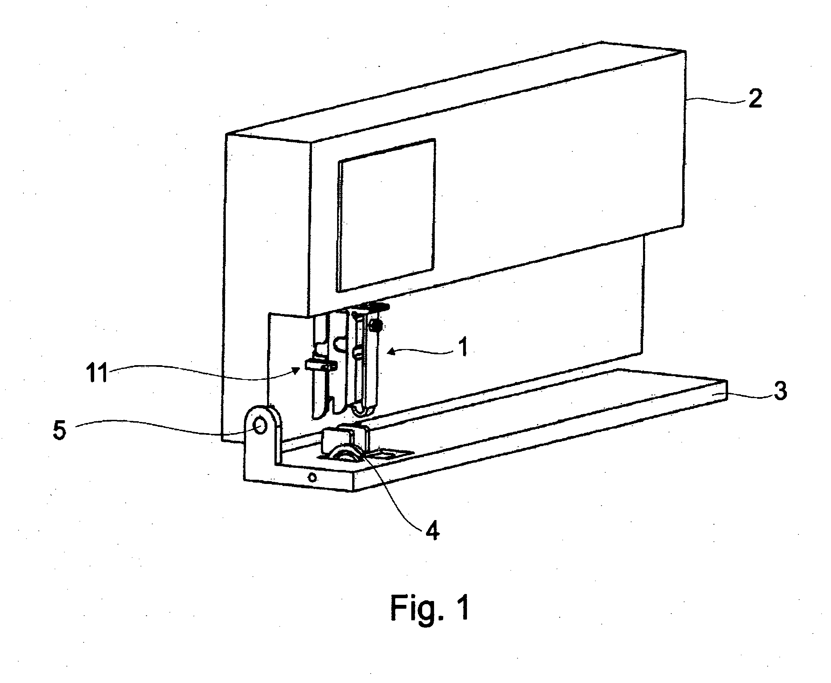

Die in der Figur 1 gezeigte Infusionsschlauchklemme 1 ist in diesem Ausführungsbeispiel vertikal ausgerichtet in einer Infusionspumpe 2 mit geöffneter Pumpenklappe 3 platziert. An der Pumpenklappe 3 befindet sich ein Schließriegel 4 der Pumpenklappe 3, wobei die Pumpenklappe 3 schwenkbar um eine Drehnase 5 der Infusionspumpe 2 gelagert ist.

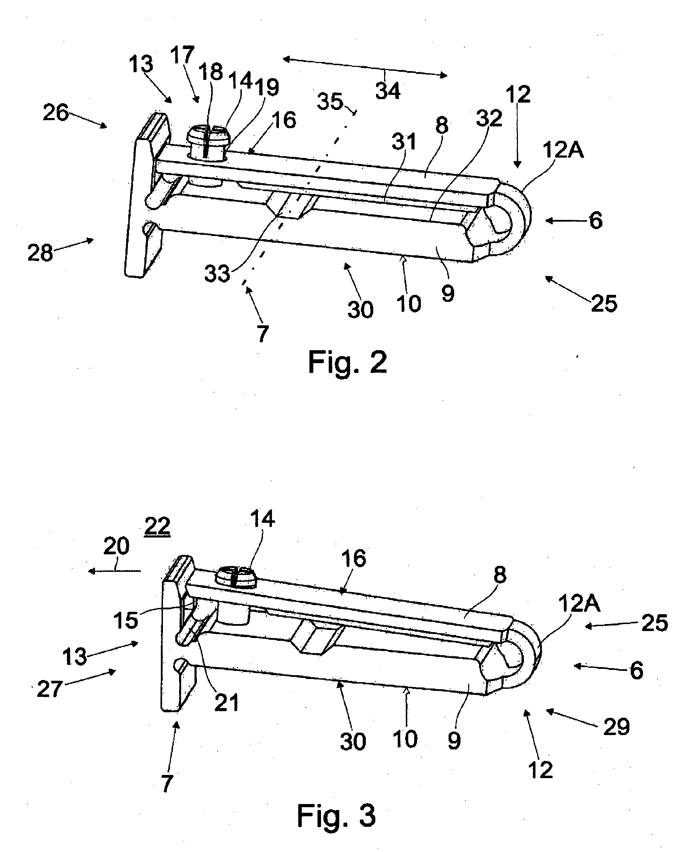

Die Infusionsschlauchklemme 1 (siehe insbesondere Figuren 2 und 3) umfasst einen Grundkörper 6 mit einem Infusionspumpenaufnahmebereich 7, mit einem ersten Klemmschenkel 8 sowie mit einem zweiten Klemmschenkel 9.

Der Infusionspumpenaufnahmebereich 7 befindet sich an der Rückseite 10 des zweiten Klemmschenkels 9, sodass die Infusionsschlauchklemme 1 passgenau in einem entsprechend ausgestalteten Sitz 11 der Infusionspumpe 2 befestigbar ist.

Die Infusionsschlauchklemme 1 ist einteilig aufgebaut, da die beiden Klemmschenkel 8 und 9 an einem ersten Infusionsschlauchklemmenende 12 mittels einer Lasche 12A untrennbar aber schwenkbar miteinander verbunden sind.

Die Infusionsschlauchklemme 1 zeichnet sich des Weiteren durch eine Schnappeinrichtung 13 zum sicheren Positionieren der beiden Klemmschenkel 8 und 9 in mehr als einer Lage zueinander aus, wobei die Schnappeinrichtung 13 hierzu ein unlösbares, erstes Rastelement 14 und ein lösbares, zweites Rastelement 15 (siehe Figur 3) aufweist, welche mit einem Schnappelement 16 der Schnappeinrichtung 13 wahlweise verrastbar sind.

Während die beiden Rastelemente 14 und 15 an dem zweiten Klemmschenkel 9 ausgestaltet sind, ist das Schnappelement 16 in diesem Ausführungsbeispiel unmittelbar von dem ersten Klemmschenkel 8 realisiert.

Das erste Rastelement 14 ist durch einen gekröpften Steg 17 gebildet, der sich lotrecht vom zweiten Klemmschenkel 9 ausgehend erstreckt und einen geschlitzten Kopfbereich 18 aufweist. Im Bereich des ersten Rastelements 14 weist das Schnappelement 16 eine Materialausnehmung 19 auf, durch welche der gekröpfte Steg 17 derart hindurch gesteckt ist, dass das Schnappelement 16 unlösbar mit dem ersten Rastelement 14 verrastet ist.

Das zweite Rastelement 15 ist eine in Auslenkrichtung 20 auslenkbare Rastnase 21, die kopfseitig 22 des Schnappelements 16 angeordnet ist. Werden die beiden Klemmschenkel 8 und 9 weiter aufeinander zubewegt, kann das Schnappelement 16 auch die auslenkbare Rastnase 21 passieren, wobei die Infusionsschlauchklemme 1 den Infusionsschlauch zusammen quetscht.

Gemäß den Darstellungen nach den Figuren 2 und 3 befindet sich die Infusionsschlauchklemme 1 in einem Betriebszustand 25 hinsichtlich einer Quetschstellung 26 (Figur 2) und hinsichtlich einer Öffenstellung 27 (Figur 3).

In der Öffenstellung 27 ist das Schnappelement 16 gemäß einer ersten Einschnapplage 28 der beiden Klemmschenkel 8 und 9 an dem ersten Rastelement 14 verrastet. Hierbei schlägt das Schnappelement 16 an dem gekröpften Kopfbereich 18 an, wobei die beiden Klemmschenkel 8 und 9 mittels einer von der Lasche 12A aufgebrachten Federkraft stets auseinander streben.

In der Quetschstellung 26 ist das Schnappelement 16 gemäß einer zweiten Einschnapplage 29 der beiden Klemmschenkel 8 und 9 an dem zweiten Rastelement 15 verrastet, sodass ein hier nicht gezeigter Infusionsschlauch mittels einer Quetscheinrichtung 30 abgequetscht ist.

Die Quetscheinrichtung 30 bildet an dem ersten Klemmschenkel 8 eine Klemmkante 31 und an dem zweiten Klemmschenkel 9 eine Klemmkantenauflage 32 aus.

Darüber hinaus weist die Infusionsschlauchklemme 1 eine radial allseits geschlossene Schlauchführungsraum 33 auf, die im Wesentlichen durch die beiden Klemmschenkel 8, 9, die Lasche 12A und dem ersten Rastelement 14 der Schnappeinrichtung 13 ausgestaltet ist. Hierbei verläuft quer zur Längserstreckung 34 der beiden Klemmschenkel 8 und 9 eine Schlauchführungsrichtung 35.

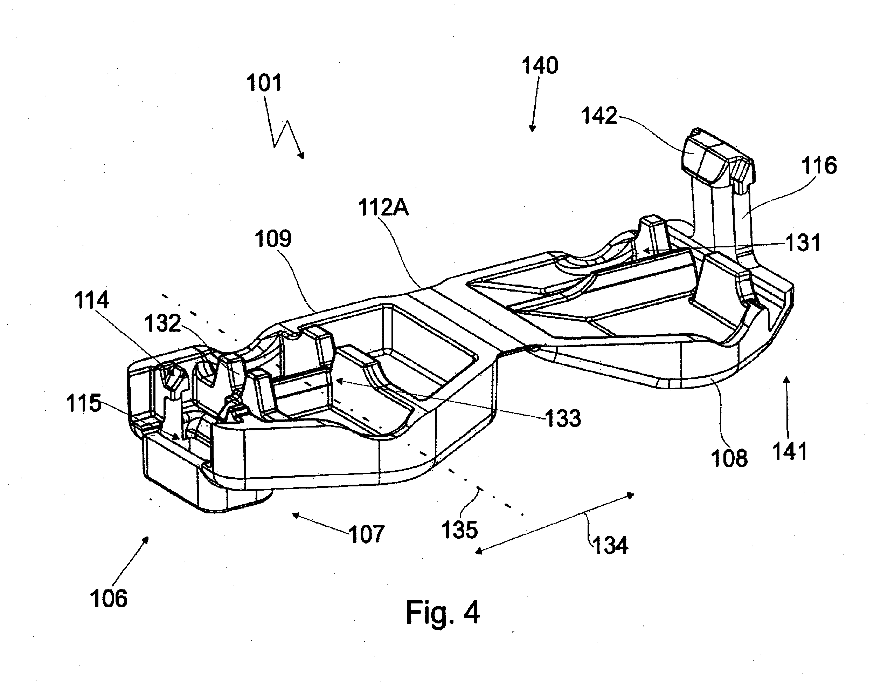

Das in den Figuren 4 bis 6 gezeigte weitere Ausführungsbeispiel einer Infusionsschlauchklemme 101 weist einen Grundkörper 106 als Spritzgußteil auf, der einen ersten Klemmschenkel 108 und einen zweiten Klemmschenkel 109 umfasst sowie einen Infusionspumpenaufnahmebereich 107.

Die beiden Klemmschenkel 108 und 109 sind an einem ersten Infusionsschlauchklemmenende 112 mittels einer federnden Lasche 112A fest aber schwenkbar miteinander verbunden.

Gemäß der Darstellung nach der Figur 4 ist die Infusionsschlauchklemme 101 in einem Herstellungszustand 140 gestreckt dargestellt, in welchem die Schnappeinrichtung 113 noch nicht mit ihrem Schnappelement 116 an einem der beiden Rastelemente 114 bzw. 115 verrastet ist. Insofern kann ein Infusionsschlauch (hier nicht gezeigt) in Schlauchführungsrichtung 135, welche quer zur Längserstreckung 134 der beiden Klemmschenkel 108 und 109 verläuft, leicht in den Schlauchführungsraum 133 der noch offenen Infusionsschlauchklemme 101 eingelegt werden.

Das erste Rastelement 114 ist hierbei unlösbar und das zweite Rastelement 115 ist hierbei lösbar mit dem Schnappelement 116 verrastbar ausgestaltet, wobei das erste Rastelement 114 entsprechend massiv ausgestaltet ist, um nicht oder nur vernachlässigbar gering ausgelenkt werden zu können.

Die Schnappeinrichtung 113 weist neben den Rastelementen 114 und 115 somit noch ein Schnappelement 116, welches mit den beiden Rastelementen 114 und 115 korrespondieren kann. Das Schnappelement 116 steht am Ende 141 des ersten Klemmschenkels 108 in etwa senkrecht von diesem ab und weist eine Schnappnase 142 auf, mittels welcher es an dem jeweiligen Rastelement 114 bzw. 115 einschnappen bzw. einrasten kann. Hierbei weist das Schnappelement 116 eine zumindest ausreichende Biegbarkeit auf, um mit der Schnappnase 142 an dem jeweiligen Rastelement 114 bzw. 115 leichter verrasten zu können.

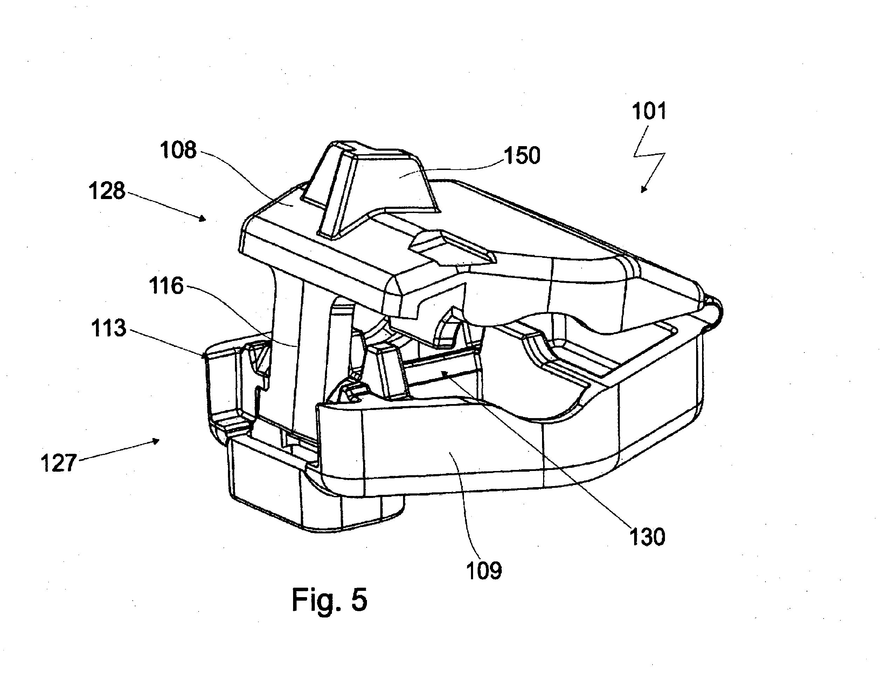

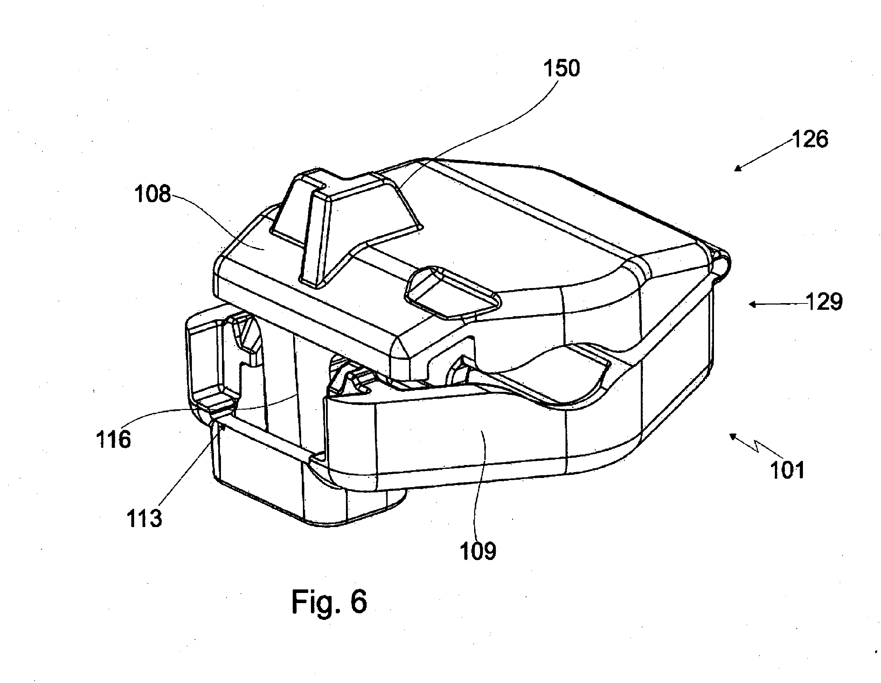

Gemäß den Darstellungen der Figuren 5 und 6 ist die Infusionsschlauchklemme 101 in einem Betriebszustand 125 gezeigt.

Hierbei befindet sich die Infusionsschlauchklemme 101 in einer Öffenstellung 127 (siehe Figur 5), bei welcher das Schnappelement 116 mit dem ersten Rastelement 114 verrastet ist. Die Infusionsschlauchklemme 101 weist insofern eine erste Einschnapplage 128 auf.

Ist das Schnappelement 116 hingegen mit dem zweiten Rastelement 115 verrastet, befindet sich die Infusionsschlauchklemme 101 in einer Quetschstellung 126 (siehe Figur 6), in welcher die Infusionsschlauchklemme 101 bzw. die beiden Klemmschenkel 108 und 109 eine zweite Einschnapplage 129 einnehmen.

Die Infusionsschlauchklemme 101 umfasst eine Quetscheinrichtung 130 mit einer Quetschkante 131 und einer Quetschkantenauflage 132.

Darüber hinaus ist an dem ersten Klemmschenkel 108 eine Betätigungsflanke 150 für einen Infusionspumpenriegel (siehe Figur 1, Bezugszeichen 4) vorgesehen.

Es versteht sich, dass es sich bei den vorstehend erläuterten Ausführungsbeispielen lediglich um erste Ausgestaltungen der erfindungsgemäßen Infusionsschlauchklemme handelt. Insofern beschränkt sich die Ausgestaltung der Erfindung nicht auf diese Ausführungsbeispiele.

Sämtliche in den Anmeldungsunterlagen offenbarten Merkmale werden als erfindungswesentlich beansprucht, sofern sie einzeln oder in Kombination gegenüber dem Stand der Technik neu sind.

### Bezugszeichenliste

- 1: Infusionsschlauchklemme
- 2: Infusionspumpe
- 3: Pumpenklappe
- 4: Schließriegel
- 5: Drehnase
- 6: Grundkörper
- 7: Infusionspumpenaufnahmebereich
- 8: erster Klemmschenkel
- 9: zweiter Klemmschenkel
- 10: Rückseite
- 11: Sitz
- 12: erstes Infusionsschlauchklemmenende
- 12A: Lasche
- 13: Schnappeinrichtung
- 14: unlösbares, erstes Rastelement
- 15: lösbares, zweites Rastelement
- 16: Schnappelement
- 17: gekröpfter Steg
- 18: geschlitzter Kopfbereich
- 19: Materialausnehmung
- 20: Auslenkrichtung
- 21: auslenkbare Rastnase
- 22: kopfseitig
- 25: Betriebszustand
- 26: Quetschstellung
- 27: Öffenstellung
- 28: erste Einschnapplage
- 29: zweite Einschnapplage
- 30: Quetscheinrichtung
- 31: Quetschkante
- 32: Quetschkantenauflage
- 33: Schlauchführungsraum
- 34: Längserstreckung
- 35: Schlauchführungsrichtung

- 101: Infusionsschlauchklemme
- 106: Grundkörper
- 107: Infusionspumpenaufnahmebereich
- 108: erster Klemmschenkel
- 109: zweiter Klemmschenkel
- 112: erstes Infusionsschlauchklemmenende
- 112A: Lasche
- 113: Schnappeinrichtung
- 114: unlösbares, erstes Rastelement
- 115: lösbares, zweites Rastelement
- 116: Schnappelement
- 125: Betriebszustand
- 126: Quetschstellung
- 127: Öffenstellung
- 128: erste Einschnapplage
- 129: zweite Einschnapplage
- 130: Quetscheinrichtung
- 131: Quetschkante
- 132: Quetschkantenauflage
- 133: Schlauchführungsraum
- 134: Längserstreckung
- 135: Schlauchführungsrichtung
- 140: Herstellungszustand
- 141: Ende
- 142: Schnappnase
- 150: Betätigungsflanke

## Patentansprüche

1. Infusionsschlauchklemme (1; 101) zum Einsetzen in eine Infusionspumpe (2) umfassend einen Grundkörper (6; 106) mit einem Infusionspumpenaufnahmebereich (7; 107), mit einem ersten Klemmschenkel (8; 108) sowie mit einem zweiten Klemmschenkel (9; 109) zum Quetschen eines Infusionsschlauches, wobei die beiden Klemmschenkel (8, 9; 108, 109) an einem ersten Infusionsschlauchklemmenende (12; 112) schwenkbar miteinander verbunden sind, **gekennzeichnet durch**
eine Schnappeinrichtung (13; 113) zum sicheren Positionieren der beiden Klemmschenkel (8, 9; 108, 109) in mehr als einer Lage (28, 29; 128, 129) zueinander, wobei die Schnappeinrichtung (13; 113) ein unlösbares Rastelement (14; 114), welches von einem Schnappelement (16, 116) nur einmal in eine erste Richtung passierbar ist, und ein lösbares Rastelement (15; 115), welches von einem Schnappelement (16, 116) sowohl in die erste Richtung als auch in eine der ersten Richtung entgegengesetzten zweiten Richtung passierbar ist, aufweist.

2. Infusionsschlauchklemme (1; 101) nach Anspruch 1,
**dadurch gekennzeichnet, dass**
die Schnappeinrichtung (13; 113) einen Betriebszustand (25; 125) mit einer Öffenstellung (27; 127) und mit einer Quetschstellung (26; 126) aufweist, bei welcher wenigstens ein Schnappelement (16; 116) der Schnappeinrichtung (13; 113) in der Öffenstellung (27; 127) an einem ersten Rastelement (14; 114) und in der Quetschstellung (26; 126) an einem zweiten Rastelement (15; 115) eingerastet anordenbar ist.

3. Infusionsschlauchklemme (1; 101) nach einem der Ansprüche 1 oder 2,
**dadurch gekennzeichnet, dass**
die Schnappeinrichtung (13; 113) zwei in Reihe geschaltete Rastelemente (14, 15; 114, 115) aufweist.

4. Infusionsschlauchklemme (1; 101) nach einem der Ansprüche 1 bis 3,
**dadurch gekennzeichnet, dass**
die Schnappeinrichtung (13; 113) wenigstens ein dem ersten Klemmschenkel (8; 108) zugeordnetes Schnappelement (16; 116) und wenigstens zwei dem zweiten Klemmschenkel (9; 109) zugeordnete Rastelemente (14, 15; 114, 115) zum Einrasten des wenigstens einen Schnappelements (16; 116) aufweist.

5. Infusionsschlauchklemme (1; 101) nach einem der Ansprüche 1 bis 4,
**dadurch gekennzeichnet, dass**
wenigstens ein Schnappelement (16; 116) und wenigstens zwei Rastelemente (14, 15; 114, 115) der Schnappeinrichtung (13; 113) derart zueinander angeordnet sind, dass beim Einschnappen der Schnappeinrichtung (13; 113) das Schnappelement (16; 116) zuerst mit dem einen (14; 114) der beiden Rastelemente (14,15; 114, 115) verrastbar ist, bevor das Schnappelement (16; 116) mit dem anderen (15; 115) der beiden Rastelemente (14, 15; 114, 115) verrastbar ist.

6. Infusionsschlauchklemme (1; 101) nach einem der Ansprüche 1 bis 5,
**dadurch gekennzeichnet, dass**
eines der beiden Rastelemente (14, 15; 114, 115) eine Betätigungsfläche für einen Aktuator einer Infusionsschlauchklemmenbetätigung der Infusionspumpe (2) aufweist, wobei insbesondere das zweite (15; 115) der beiden Rastelemente (14, 15; 114, 115) weiter auslenkbar ausgestaltet ist als das erste (14; 114) der beiden Rastelemente (14, 15; 114, 115).

7. Infusionsschlauchklemme (1; 101) nach einem der Ansprüche 1 bis 6,
**dadurch gekennzeichnet, dass**
die Infusionsschlauchklemme (1; 101) zumindest in einem Betriebszustand (25; 125) eine radial allseits geschlossene Schlauchführungsraum (33; 133) mit einer quer zu der Längserstreckung (34; 134) der beiden Klemmschenkel (8, 9; 108, 109) angeordneten Schlauchführungsrichtung (35; 135) aufweist.

8. Infusionsschlauchklemme (1; 101) nach einem der Ansprüche 1 bis 7,
**dadurch gekennzeichnet, dass**
die lnfusionsschlauchklemme (1; 101) eine Betätigungsflanke (150) für einen Infusionspumpenriegel (4) aufweist.

9. Verfahren zum Verwenden einer Infusionsschlauchklemme (1; 101) an einer Infusionspumpe (2), bei welchem ein erster Klemmschenkel (8; 108) und ein zweiter Klemmschenkel (9; 109) der Infusionsschlauchklemme (1; 101) um einen Infusionsschlauchklemmenendbereich (12; 112) herum aufeinander zu geschwenkt werden, um hierbei einen Infusionsschlauch abquetschen zu können, **dadurch gekennzeichnet, dass**
eine Schnappeinrichtung (13; 113) mit wenigstens einem Schnappelement (16; 116) mit einem ersten Rastelement (14; 114) in eine erste Einschnapplage (28; 128) hinein verrastet wird, in welcher der Infusionsschlauch zwischen den beiden Klemmschenkeln (8, 9; 108, 109) gehalten wird bzw. ist, und dass die Schnappeinrichtung (13; 113) mit dem wenigstens einem Schnappelement (16; 116) oder mit einem weiteren Schnappelement mit einem zweiten Rastelement (15; 115) in eine zweite Einschnapplage (29; 129) hinein verrastet wird, in welcher der Infusionsschlauch abquetscht wird bzw. ist, wobei die Schnappeinrichtung (13; 113) ein unlösbares Rastelement (14; 114), welches von einem Schnappelement (16, 116) nur einmal in eine erste Richtung passierbar ist, und ein lösbares Rastelement (15; 115), welches von einem Schnappelement (16, 116) sowohl in die erste Richtung als auch in eine der ersten Richtung entgegengesetzten zweiten Richtung passierbar ist, aufweist.

10. Verfahren nach Anspruch 9,
**dadurch gekennzeichnet, dass**
zum Wiederöffnen des Infusionsschlauchs das zweite Rastelement (15; 115) derart ausgelenkt wird, dass die Schnappeinrichtung (13; 113) von der zweiten Einschnapplage (29; 129) in die erste Einschnapplage (28; 128) zurückkehrt.

11. Infusionspumpe (2) mit einer Aufnahme (11) für eine schlauchseitig angeordnete Infusionsschlauchklemme (1; 101) und mit einem Aktuator zum Betätigen der schlauchseitigen Infusionsschlauchklemme (1; 101),
**gekennzeichnet durch**
eine Infusionsschlauchklemme (1; 101) nach einem der vorhergehenden Ansprüche1 bis 8.

## Claims

1. An infusion tube clamp (1; 101) for being inserted in an infusion pump (2), comprising:
a base member (6; 106) including an infusion pump receiving area (7, 107), a first clamping leg (8; 108) and a second clamping leg (9; 109) for squeezing an infusion tube, wherein the two clamping legs (8, 9; 108, 109) are swivelconnected to each other at a first infusion tube clamp end (12; 112); and
**characterized by**
a click device (13; 113) for safely positioning the two clamping legs (8, 9; 108, 109) in more than one position (28, 29; 128, 129) relative to each other, wherein the click device (13; 113) includes a non-detachable detent element (14; 114) which can be passed by a click element (16; 116) only once in a first direction and a detachable detent element (15; 115) which can be passed by a click element (16; 116) both in the first direction and in a second direction opposed to the first direction.

2. The infusion tube clamp (1; 101) according to claim 1,
**characterized in that**
the click device (13; 113) has an operating state (25; 125) including an opening position (27; 127) and a squeezing position (26; 126) in which at least one click element (16; 116) of the click device (13; 113) can be arranged to be engaged in the opening position (27; 127) in a first detent element (14; 114) and in the squeezing position (26; 126) in a second detent element (15; 115).

3. The infusion tube clamp (1; 101) according to one of claims 1 or 2, **characterized in that**
the click device (13; 113) includes two detent elements (14, 15; 114, 115) that are connected in series.

4. The infusion tube clamp (1; 101) according to one of claims 1 to 3, **characterized in that**
the click device (13; 113) includes at least one click element (16; 116) that is associated with the first clamping leg (8; 108) and at least two detent elements (14, 15; 114, 115) that are associated with the second clamping leg (9; 109) for engaging the at least one click element (16; 116).

5. The infusion tube clamp (1; 101) according to one of claims 1 to 4, **characterized in that**
at least one click element (16; 116) and at least two detent elements (14, 15; 114, 115) of the click device (13; 113) are arranged relative to each other so that upon snap-in of the click device (13; 113), the click element (16; 116) is first engageable in one (14; 114) of the two detent elements (14, 15; 114, 115), before the click element (16; 116) is engageable in the other one of the two detent elements (14, 15; 114, 115).

6. The infusion tube clamp (1; 101) according to one of claims 1 to 5, **characterized in that**
one of the two detent elements (14, 15; 114, 115) has an actuating surface for an actuator of an infusion tube clamp actuation of the infusion pump (2), wherein in particular the second one (15; 115) of the two detent elements (14, 15; 114, 115) is configured to be deflectable further than the first one (14; 114) of the two detent elements (14, 15; 114, 115).

7. The infusion tube clamp (1; 101) according to one of claims 1 to 6, **characterized in that**
the infusion tube clamp (1; 101) has in at least one operating state (25; 125) a tube guiding space (33; 133) being radially closed on all sides and having a tube guiding direction (35; 135) arranged transversely to the longitudinal extension (34; 134) of the two clamping legs (8, 9; 108, 109).

8. The infusion tube clamp (1; 101) according to one of claims 1 to 7, **characterized in that**
the infusion tube clamp (1; 101) includes an operating flank (150) for an infusion pump locking bar (4).

9. A method of using an infusion tube clamp (1; 101) at an infusion pump (2) in which a first clamping leg (8; 108) and a second clamping leg (9; 109) of the infusion tube clamp (1; 101) are swiveled toward each other around an infusion tube clamp end area (12; 112) so that an infusion tube can be squeezed, **characterized in that**
a click device (13; 113) is engaged by at least one click element (16; 116) with a first detent element (14; 114) into a first snap-in position (28; 128) in which the infusion tube is retained between the two clamping legs (8, 9; 108, 109), and the click device (13; 113) is engaged by the at least one click element (16; 116) or by another click element (16; 116) with a second detent element (15; 115) into a second snap-in position (29; 129) in which the infusion tube is squeezed, wherein the click device (13; 113) includes a non-detachable detent element (14; 114) that can be passed by a click element (16; 116) only once in a first direction and a detachable detent element (15; 115) that can be passed by a click element (16; 116) both in the first direction and in a second direction opposed to the first direction.

10. The method according to claim 9,
**characterized in that**
for re-opening the infusion tube, the second detent element (15; 115) is deflected so that the click device (13; 113) returns from the second snap-in position (29; 129) to the first snap-in position (28; 128).

11. An infusion pump (2) including a seat (11) for an infusion tube clamp (1; 101) arranged on a tube side and an actuator for actuating the tube-side infusion tube clamp (1; 101),
**characterized by**
an infusion tube clamp (1; 101) according to one of the preceding claims 1 to 8.

## Revendications

1. Pince de serrage (1 ; 101) pour tuyau flexible de perfusion destinée à être montée dans une pompe à perfusion (2), comportant un corps de base (6 ; 106) avec une zone de réception (7 ; 107) pour la pompe à perfusion, avec une première branche de serrage (8 ; 108) et avec une deuxième branche de serrage (9 ; 109) pour comprimer un tuyau flexible de perfusion, les deux branches de serrage (8, 9 ; 108, 109) étant reliées l'une à l'autre de manière pivotante sur une première extrémité (12 ; 112) de ladite pince de serrage,
**caractérisée par**
un dispositif d'encliquetage (13 ; 113) pour positionner de manière sûre les deux branches de serrage (8, 9 ; 108, 109) l'une par rapport à l'autre dans plus d'une position (28, 29; 128, 129), le dispositif d'encliquetage (13; 113) comportant un élément de blocage (14; 114) inamovible qui peut être franchi par un élément d'encliquetage (16, 116) seulement une fois dans une première direction, et un élément de blocage (15; 115) amovible qui peut être franchi par un élément d'encliquetage (16, 116) tant dans la première direction que dans une deuxième direction opposée à la première direction.

2. Pince de serrage (1 ; 101) pour tuyau flexible de perfusion selon la revendication 1, **caractérisée en ce que** le dispositif d'encliquetage (13 ; 113) comporte une position de service (25 ; 125) avec une position d'ouverture (27 ; 127) et avec une position de serrage (26 ; 126), dans laquelle au moins un élément d'encliquetage (16 ; 116) du dispositif d'encliquetage (13 ; 113) peut être disposé de manière encliquetée dans la position d'ouverture (27 ; 127) sur un premier élément de blocage (14; 114) et dans la position de serrage (26 ; 126) sur un deuxième élément de blocage (15 ; 115).

3. Pince de serrage (1 ; 101) pour tuyau flexible de perfusion selon la revendication 1 ou 2, **caractérisée en ce que** le dispositif d'encliquetage (13 ; 113) comporte deux éléments de blocage (14, 15 ; 114, 115) montés en série.

4. Pince de serrage (1 ; 101) pour tuyau flexible de perfusion selon l'une quelconque des revendications 1 à 3, **caractérisée en ce que** le dispositif d'encliquetage (13 ; 113) comporte au moins un élément d'encliquetage (16 ; 116) associé à la première branche de serrage (8 ; 108) et au moins deux éléments de blocage (14, 15 ; 114, 115) associés à la deuxième branche de serrage (9 ; 109) pour l'encliquetage dudit au moins un élément d'encliquetage (16 ; 116).

5. Pince de serrage (1 ; 101) pour tuyau flexible de perfusion selon l'une quelconque des revendications 1 à 4, **caractérisée en ce qu'**au moins un élément d'encliquetage (16 ; 116) et au moins deux éléments de blocage (14, 15 ; 114, 115) du dispositif d'encliquetage (13 ; 113) sont disposés l'un par rapport à l'autre de telle sorte qu'au moment de l'encliquetage du dispositif d'encliquetage (13; 113), l'élément d'encliquetage (16 ; 116) peut être enclenché d'abord avec l'un des deux éléments de blocage (14, 15; 114, 115) avant que l'élément d'encliquetage (16; 116) ne puisse être enclenché avec l'autre (15 ; 115) des deux éléments de blocage (14, 15 ; 114, 115).

6. Pince de serrage (1 ; 101) pour tuyau flexible de perfusion selon l'une quelconque des revendications 1 à 5, **caractérisée en ce que** l'un des deux éléments de blocage (14, 15; 114, 115) comporte une surface d'actionnement pour un actionneur d'un dispositif d'actionnement de la pince de serrage pour tuyau flexible de perfusion de la pompe à perfusion (2), en particulier le deuxième (15 ; 115) des deux éléments de blocage (14, 15 ; 114, 115) étant configuré de manière à pouvoir dévier davantage que le premier (14 ; 114) des deux éléments de blocage (14, 15 ; 114, 115).

7. Pince de serrage (1 ; 101) pour tuyau flexible de perfusion selon l'une quelconque des revendications 1 à 6, **caractérisée en ce que** ladite pince de serrage (1 ; 101) comporte, au moins dans une position de service (25 ; 125), un espace de guidage du tuyau flexible (33 ; 133), fermé radialement de toutes parts, avec une direction de guidage du tuyau flexible (35 ; 135) disposée transversalement à l'étendue longitudinale (34 ; 134) des deux branches de serrage (8, 9 ; 108, 109).

8. Pince de serrage (1 ; 101) pour tuyau flexible de perfusion selon l'une quelconque des revendications 1 à 7, **caractérisée en ce que** ladite pince de serrage (1 ; 101) comporte un flanc d'actionnement (150) pour un verrou (4) de la pompe à perfusion.

9. Procédé permettant l'utilisation d'une pince de serrage (1 ; 101) pour tuyau flexible de perfusion sur une pompe à perfusion (2), dans lequel une première branche de serrage (8 ; 108) et une deuxième branche de serrage (9 ; 109) de la pince de serrage (1 ; 101) sont amenées à pivoter l'une sur l'autre autour d'une zone d'extrémité (12; 112) de la pince de serrage afin de pouvoir comprimer à cette occasion un tuyau flexible de perfusion,
**caractérisé en ce que**
un dispositif d'encliquetage (13; 113) avec au moins un élément d'encliquetage (16; 116) muni d'un premier élément de blocage (14; 114) est enclenché dans une première position d'encliquetage (28; 128), dans laquelle le tuyau flexible de perfusion sera ou est maintenu entre les deux branches de serrage (8, 9 ; 108, 109), et **en ce que** le dispositif d'encliquetage (13 ; 113) avec ledit au moins un élément d'encliquetage (16 ; 116) ou avec un autre élément d'encliquetage muni d'un deuxième élément de blocage (15; 115) est enclenché dans une deuxième position d'encliquetage (29 ; 129), dans laquelle le tuyau flexible de perfusion sera ou est comprimé, le dispositif d'encliquetage (13 ; 113) comportant un élément de blocage (14; 114) inamovible qui peut être franchi par un élément d'encliquetage (16, 116) seulement une fois dans une première direction, et un élément de blocage (15; 115) amovible qui peut être franchi par un élément d'encliquetage (16, 116) tant dans la première direction que dans une deuxième direction opposée à la première direction.

10. Procédé selon la revendication 9, **caractérisé en ce que** pour la réouverture du tuyau flexible de perfusion, le deuxième élément de blocage (15 ; 115) est dévié de telle sorte que le dispositif d'encliquetage (13 ; 113) est ramené de la deuxième position d'encliquetage (29 ; 129) dans la première position d'encliquetage (28 ; 128).

11. Pompe à perfusion (2) comportant un logement (11) pour une pince de serrage (1 ; 101) pour tuyau flexible de perfusion et comportant un actionneur pour actionner ladite pince de serrage (1 ; 101) du côté du tuyau flexible, **caractérisée par** une pince de serrage (1 ; 101) pour tuyau flexible de perfusion selon l'une quelconque des revendications précédentes 1 à 8.
